Europäisches Patentamt

European Patent Office

Office européen des brevets

(11) Numéro de publication : **0 327 431 B1**

(19)

# FASCICULE DE BREVET EUROPEEN

(12)

(45) Date de publication du fascicule du brevet :
16.12.92 Bulletin 92/51

(51) Int. Cl.⁵ : **A61M 25/00,** A61M 29/02

(21) Numéro de dépôt : **89400223.7**

(22) Date de dépôt : **26.01.89**

(54) **Sonde destinée à être introduite à l'intérieur d'un corps vivant.**

(30) Priorité : **01.02.88 FR 8801120**

(43) Date de publication de la demande :
**09.08.89 Bulletin 89/32**

(45) Mention de la délivrance du brevet :
**16.12.92 Bulletin 92/51**

(84) Etats contractants désignés :
**AT BE CH DE ES FR GB GR IT LI LU NL SE**

(56) Documents cités :
**WO-A-84/01513**
**WO-A-88/00071**
**US-A- 4 299 237**
**US-A- 4 423 725**
**US-A- 4 581 017**

(73) Titulaire : **Boussignac, Georges**
**1, Allée de Provence**
**F-92160 Antony (FR)**
Titulaire : **Labrune, Jean-Claude**
**19a rue Massenet**
**F-92310 Sèvres (FR)**

(72) Inventeur : **Boussignac, Georges**
**1, Allée de Provence**
**F-92160 Antony (FR)**
Inventeur : **Labrune, Jean-Claude**
**19a rue Massenet**
**F-92310 Sèvres (FR)**

(74) Mandataire : **Bonnetat, Christian et al**
**CABINET BONNETAT 23, Rue de Léningrad**
**F-75008 Paris (FR)**

EP 0 327 431 B1

Jouve, 18, rue Saint-Denis, 75001 PARIS

**Description**

La présente invention concerne une sonde destinée à être introduite à l'intérieur d'un corps vivant, du type comprenant une structure longiforme apte à être introduite dans un canal dudit corps vivant transportant un fluide, et présentant, à son extrémité destinée à être introduite dans le canal, une capacité unique, liée à ladite structure, entourant celle-ci et radialement déformable de façon à pouvoir venir au contact de la paroi interne dudit canal.

De telles sondes à ballonet, ou cathéters du type dit "à dilatation", permettent une intervention au sein d'un canal corporel, notamment un vaisseau sanguin, pour traiter les désordres qui y ont été constatés. De tels désordres sont généralement constitués par des dépôts, athéromes, sur les parois du canal, entraînant des rétrécissements, sténoses, de ce dernier.

Plus particulièrement, de telles sondes permettent, d'une part, grâce à la capacité radialement déformable, d'améliorer la section de passage du canal, et, d'autre part, par injection de produit de traitement, par exemple à travers les parois de ladite capacité, de rendre durable ce rétablissement de la section de passage du canal, en formant éventuellement "in situ" une prothèse de section correspondante.

Cependant, il est nécessaire de maintenir, pendant l'intervention, une circulation minimale du fluide dans le canal corporel afin de ne pas risquer d'endommager les tissus ou organes situés en aval de la capacité, si le canal est obturé par la sonde. Jusqu'à présent, une telle circulation de fluide est assurée, soit par perfusion de fluide amené de l'extérieur du corps par un conduit prévu dans ladite structure longiforme de la sonde, soit, comme cela est montré par le brevet US-A-4 581 017, par "perfusion interne", des orifices latéraux débouchant dans le canal étant prévus dans ladite structure en amont et en aval de ladite capacité et étant reliés l'un à l'autre par une "dérivation" traversant ladite structure.

La première solution n'est pas entièrement satisfaisante car elle exige l'amenée d'un fluide "extérieur" au corps. Par ailleurs, dans les deux cas indiqués ci-dessus, au moins une partie de la section, nécessairement faible, de la structure longiforme est utilisée pour le passage du fluide, ce qui, bien entendu, rend plus difficile, sinon impossible, le passage, dans ladite structure, de moyens de guidage de la sonde, ou éventuellement d'instruments endoscopiques ou chirurgicaux, ou la mise en place des moyens assurant la déformation de ladite capacité.

Par ailleurs, le document WO-A-88/00071 décrit une sonde destinée à être introduite à l'intérieur d'un corps vivant, du type comprenant une structure longiforme apte à être introduite dans un canal dudit corps vivant transportant un fluide, et présentant, à son extrémité destinée à être introduite dans le canal, une capacité unique liée à ladite structure, entourant celle-ci et radialement déformable de façon à pouvoir venir au contact de la paroi interne dudit canal, ladite sonde comprenant un passage lié à ladite structure, s'étendant de façon au moins sensiblement parallèle à ladite structure et à l'extérieur de cette dernière, sur une distance correspondant sensiblement à la dimension longitudinale de ladite capacité de façon à pouvoir mettre en communication les parties dudit canal immédiatement en amont et en aval de ladite capacité, pour ainsi maintenir une circulation minimale de fluide dans ledit canal.

Cependant, la sonde décrite dans ce dernier document ne permet pas d'introduire et de maintenir des instruments endoscopiques ou chirurgicaux tels qu'une fibre optique, coaxialement audit canal, ni d'exercer une pression régulièrement répartie autour de l'axe de la sonde.

La présente invention a pour objet d'éviter ces inconvénients, et concerne une sonde, du type indiqué dans le document WO-A-88/00071, permettant de maintenir une fibre optique axiale et d'exercer une pression régulièrement répartie autour de l'axe de la sonde tout en assurant une communication suffisante pour le fluide entre les parties amont et aval du ballonnet.

A cet effet, la sonde, du type indiqué ci-dessus, est remarquable, selon l'invention, en ce que ledit passage est formé par une pluralité de conduits s'étendant au moins sensiblement parallèlement l'un par rapport à l'autre et en ce que lesdits conduits sont angulairement équi-répartis autour de l'axe longitudinal de ladite structure.

De préférence, les axes desdits conduits s'étendent sur une surface cylindrique dont l'axe longitudinal est confondu avec celui de ladite structure.

Par ailleurs, ladite capacité étant gonflable, il est avantageux que lesdits conduits soient fermés dans l'état dégonflé de ladite capacité, et soient ouverts dans l'état gonflé de cette dernière.

Ladite capacité peut être à double paroi, et lesdits conduits peuvent passer à l'intérieur de cette double paroi. Dans ce cas, il est avantageux que la paroi extérieure de ladite double paroi soit au moins partiellement poreuse sous pression, tandis que la paroi intérieure de ladite double paroi forme un ballonnet gonflable.

La partie centrale de ladite paroi extérieure peut être poreuse sous pression, tandis que les extrémités de ladite paroi extérieure sont préformées pour présenter la forme de bourrelets. La sonde comporte alors avantageusement un conduit pour alimenter ladite double paroi en produit susceptible de traverser ladite partie centrale de la paroi extérieure.

Les figures du dessin annexé feront bien comprendre comment l'invention peut être réalisée. Sur ces figures, des références identiques désignent des éléments semblables.

La figure 1 est une vue schématique en coupe longitudinale de la sonde selon l'invention, dont la capacité déformable est dans l'état dégonflé.

La figure 2 est une vue schématique semblable à la figure 1, la capacité déformable étant dans l'état gonflé.

La figure 3 est une vue en coupe transversale selon la ligne III-III de la figure 2.

La figure 4 illustre une variante de réalisation de la sonde conforme à l'invention, à l'état gonflé.

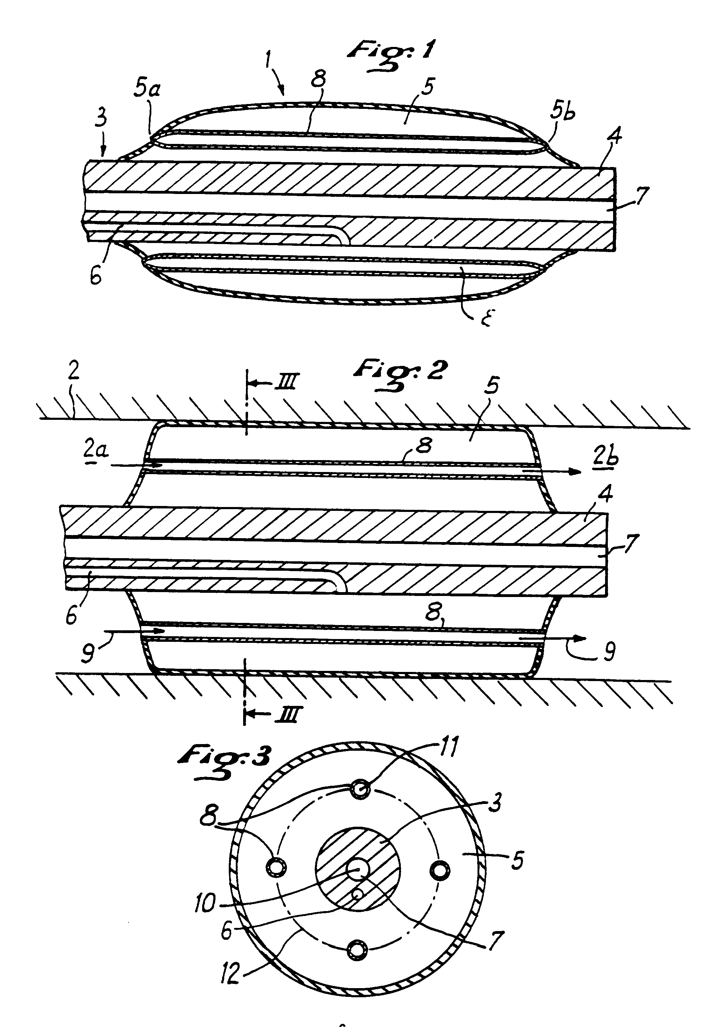

La sonde 1, destinée à être introduite dans un canal corporel 2, par exemple un vaisseau sanguin, tel qu'une artère, comporte une structure longiforme 3 dont seule l'extrémité 4 destinée à être introduite dans le canal 2 est représentée sur le dessin, ladite extrémité 4 présentant une capacité 5, liée à la structure 3, radialement déformable, notamment gonflable par des moyens de pression (non représentés) reliés à la capacité 5 par un tube 6 s'étendant dans la structure 3. Dans l'état gonflé, la capacité 5 vient au contact de la paroi interne du canal 2, comme le montre la figure 2, en rétablissant, éventuellement, la section normale du canal.

De plus, la structure 3 présente une lumière 7 pour le passage, par exemple, de moyens de guidage (non représentés).

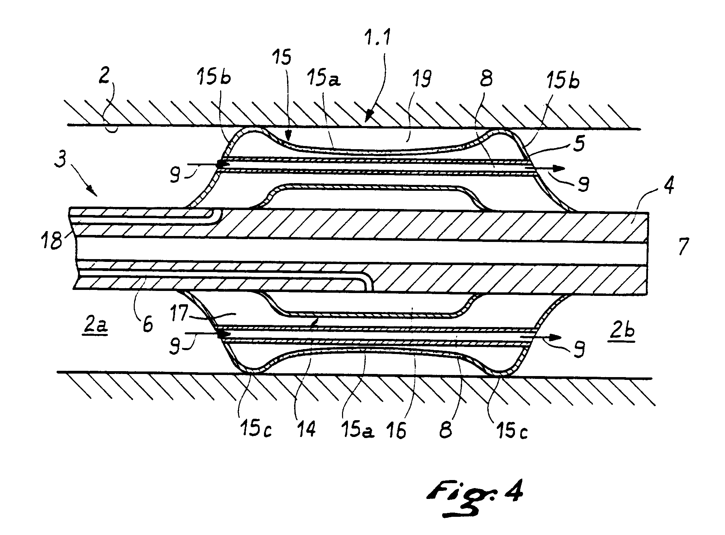

Selon l'invention, une pluralité de conduits 8 (quatre dans l'exemple représenté sur la figure 3) traversent la capacité 5, en s'étendant de façon au moins sensiblement parallèle à la structure 3, pour mettre en communication la partie 2a du canal 2 immédiatement en amont de la capacité 5 et la partie 2b du canal 2 immédiatement en aval de ladite capacité, en assurant ainsi une circulation minimale de fluide (flèches 9) pendant l'intervention (figure 2).

Comme le montre la figure 3, les conduits 8 sont avantageusement angulairement équi-répartis autour de l'axe longitudinal 10 de la structure, pour assurer ainsi une meilleure répartition du fluide dans la zone aval 2b du canal 2. En particulier, les axes 11 des conduits 8 s'étendent sur une surface cylindrique (de trace 12 sur la figure 3) dont l'axe longitudinal est confondu avec l'axe 10 de la structure 3.

Par ailleurs, les conduits 8, ouverts dans l'état gonflé de la capacité 5 (figure 2), peuvent être fermés au moins partiellement dans l'état dégonflé de cette dernière (figure 1) du fait de l'"affaissement" des extrémités 5a,5b de la capacité 5 (figure 1).

Le conduit central 7 peut recevoir un guide permettant l'implantation dans la partie lésée, une fibre optique permettant la vision de la partie lésée, ou une fibre optique pour le tir au laser, l'émetteur d'onde étant relié à l'autre extrémité de la sonde. L'extrémité distale de la sonde au niveau de la capacité 5 peut comporter des moyens opaques aux rayons X, permettant de repérer le déplacement de l'extrémité opérationnelle de la sonde en son positionnement correct. L'extrémité proximale de la sonde peut être reliée à un dispositif de recirculation du fluide biologique sous pression tel que le sang, destiné à l'irrigation des organes desservis par le vaisseau en cours de traitement. La sonde sera, dans ce cas, munie à son extrémité distale d'un détendeur de pression qui empêchera les risques de lésion par le fluide sous pression. Ce détendeur reprendra le principe d'une chambre de décompression et sera généralement constitué par une gaine souple en latex, par exemple, intégrée à l'extrémité de la sonde. Il éliminera les risques de traumatisme provoqués par la pression du fluide biologique de recirculation en sortie de sonde et est souhaitable dans ce type de sonde, lors de son installation et de sa mise en place dans des vaisseaux desservant des organes sensibles à l'hypoxie (artères coronaires et cerveau).

La structure 3 peut être réalisée en matière plastique, telle qu'un polychlorure de vinyle, un polytétrafluoroéthylène, un polyéthylène. Elle pourra, sur une partie ou en totalité, être constituée d'un fil d'acier bobiné en hélice à spires jointives et être revêtue extérieurement ou intérieurement de polytétrafluoroéthylène. Les conduits 8 peuvent également être réalisés en matière plastique. La capacité 5 sera généralement réalisée en matière plastique souple et élastiquement déformable pour permettre l'accroissement de son volume. Elle sera préformée à la dimension et/ou à la forme souhaitée.

La structure 3 peut être fabriquée par les procédés classiques d'extrusion. Il pourra en être de même pour les conduits 8 alors que la capacité 5 sera généralement obtenue par moulage. On pourra également fabriquer en une seule pièce par moulage ou par un procédé de fabrication de même nature, les conduits 8 et la capacité 5, ce procédé de fabrication étant souhaitable pour des motifs de sécurité. On pourra utiliser également les apports de l'exposition aux rayons pour obtenir les résistances et les déformabilités souhaitées.

La variante de réalisation 1.1 de la sonde conforme à la présente invention, représentée à l'état gonflé à l'intérieur d'un canal corporel 2 sur la figure 4, comporte les éléments 3 à 8 décrits ci-dessus. Dans la sonde 1.1, la capacité 5 comporte deux parois 14 et 15. La paroi intérieure 14 est enveloppée par la paroi extérieure 15 et délimite avec la structure 3 une chambre expansible 16 dans laquelle débouche le tube 6. La paroi extérieure 15, enveloppant la paroi intérieure 14, délimite avec cette dernière une chambre 17, éventuellement reliée à l'extérieur par un canal 18. Les conduits 8 traversent ladite chambre 17. La paroi extérieure 15 peut comporter une partie centrale 15a poreuse, lorsque la chambre 16 est sous pression. Les extrémités 15b de la paroi 15 peuvent être préformées pour présenter chacune un bourrelet périphérique 15c, lesdits bourrelets périphériques 15c encadrant la partie centrale poreuse 15a.

Ainsi, la capacité 5 possède une double paroi 14, 15 et sa paroi extérieure 15 comporte une portion 15a perméable sous pression, susceptible de laisser passer vers l'extérieur, (c'est-à-dire dans l'espace 19 délimité entre le canal 2, les bourrelets 15c et la portion de paroi 15a) un produit susceptible de permettre un traitement local dudit canal, par exemple un produit cytotoxique ou cytostatique, ou bien encore une endoprothèse. Un tel fluide peut être contenu dans la chambre 17 lors de l'introduction de la sonde dans le canal 2, ou bien être apporté à celle-ci en cours d'opération par le conduit 18. On remarquera que les bourrelets d'extrémité 15c assurent une certaine étanchéité à l'espace 19.

## Revendications

1. Sonde destinée à être introduite à l'intérieur d'un corps vivant, du type comprenant une structure longiforme apte à être introduite dans un canal dudit corps vivant transportant un fluide, et présentant, à son extrémité detinée à être introduite dans le canal, une capacité unique liée à ladite structure, entourant celle-ci et radialement déformable de façon à pouvoir venir au contact de la paroi interne dudit canal, ladite sonde comprenant un passage lié à ladite structure (3), s'étendant de façon au moins sensiblement parallèle à ladite structure (3) et à l'extérieur de cette dernière, sur une distance correspondant sensiblement à la dimension longitudinale de ladite capacité (5), de façon à pouvoir mettre en communication les parties (2a,2b) dudit canal (2) immédiatement en amont et en aval de ladite capacité (5), pour ainsi maintenir une circulation minimale de fluide dans ledit canal (2),
caractérisée en ce que ledit passage est formé par une pluralité de conduits (8) s'étendant au moins sensiblement parallèlement l'un par rapport à l'autre et en ce que lesdits conduits (8) sont angulairement équi-répartis autour de l'axe longitudinal (10) de ladite structure (3).

2. Sonde selon la revendication 1,
caractérisée en ce que les axes (11) desdits conduits s'étendent sur une surface cylindrique (12) dont l'axe longitudinal (10) est confondu avec celui de ladite structure (3).

3. Sonde selon l'une des revendications 1 ou 2, dans laquelle ladite capacité est gonflable,
caractérisée en ce que lesdits conduits (8) sont fermés dans l'état dégonflé de ladite capacité (5), et sont ouverts dans l'état gonflé de cette dernière.

4. Sonde selon la revendication 1,
caractérisée en ce que ladite capacité (5) est à double paroi (14, 15) et en ce que lesdits conduits (8) passent à l'intérieur de cette double paroi.

5. Sonde selon la revendication 4,
caractérisée en ce que la paroi extérieure (15) de ladite double paroi (14,15) est au moins partiellement poreuse sous pression, tandis que la paroi intérieure (14) de ladite double paroi (14,15) forme un ballonnet gonflable.

6. Sonde selon la revendication 5,
caractérisée en ce que la partie centrale (15a) de ladite paroi extérieure (15) est poreuse sous pression, tandis que les extrémités de ladite paroi extérieure sont préformées pour présenter la forme de bourrelets (15b).

7. Sonde selon la revendication 6,
caractérisée en ce qu'elle comporte un conduit (18) pour alimenter ladite double paroi (14,15) en produit susceptible de traverser ladite partie centrale de la paroi extérieure.

## Claims

1. Probe designed to be introduced inside a living body, of the type comprising an elongate structure capable of being introduced inside a fluid-transporting channel of the said living body and having, at its end designed to be introduced inside the channel, a single volume connected to the said structure, surrounding the latter and radially deformable in such a way as to be able to come into contact with the inner wall of the said channel, the said probe comprising a passage connected to the said structure (3), extending at least substantially parallel to the said structure (3) and outside the latter, over a distance corresponding substantially to the longitudinal dimension of the said volume (5), in such a way as to be able to bring into communication the parts (2a, 2b) of the said channel (2) immediately upstream and downstream of the said volume (5), in order thereby to maintain a minimum circulation of fluid in the said channel (2), characterised in that the said passage is formed by a plurality of conduits (8) extending at least substantially parallel to one another, and in that the said conduits (8) are distributed at uniform angles around the longitudinal axis (10) of the said structure (3).

2. Probe according to Claim 1, characterised in that the axes (11) of the said conduits extend over a cylindrical surface (12) whose longitudinal axis (10) coincides with that of the said structure (3).

3. Probe according to either of Claims 1 or 2, in which the said volume is inflatable, characterised in that the said conduits (8) are closed when the said volume (5) is in the deflated state and are open when the said volume is in the inflated state.

4. Probe according to Claim 1, characterised in that the said volume (5) has a double wall (14, 15), and in that the said conduits (8) pass inside this double wall.

5. Probe according to Claim 4, characterised in that the outer wall (15) of the said double wall (14, 15) is at least partially porous under pressure, while the inner wall (14) of the said double wall (14, 15) forms an inflatable balloon.

6. Probe according to Claim 5, characterised in that the central part (15a) of the said outer wall (15) is porous under pressure, while the ends of the said outer wall are preformed so as to present the shape of flanges (15b).

7. Probe according to Claim 6, characterised in that it comprises a conduit (18) for supplying to the said double wall (14, 15) a product which is capable of passing through the said central part of the outer wall.

**Patentansprüche**

1. Sonde, bestimmt zur Einführung ins Innere eines lebenden Körpers, die eine längliche, zum Einführen in einen Kanal des lebenden Körpers geeignete Struktur umfaßt, wobei der Kanal eine Flüssigkeit transportiert, und an ihrem zum einführen in den Kanal bestimmten ende eine einzige, mit der Struktur verbundene Verdickung aufweist, die diese umgibt und radial derartig verformbar ist, daß sie mit der inneren Seitenwand des Kanals in Berührung kommen kann, wobei die Sonde einen mit der Struktur (3) verbundenen Durchlaß umfaßt, der sich wenigstens ungefähr parallel zur Struktur (3) und außerhalb von dieser auf eine ungefähr der Länge der Verdickung (5) entsprechende Distanz dergestalt erstreckt, daß er die Teile (2a, 2b) des Kanals (2) unmittelbar vor und hinter der Verdickung (5) in Verbindung bringen kann, um so eine Minimalzirkulation der Flüssigkeit im Kanal (2) aufrechtzuerhalten, dadurch gekennzeichnet, daß der Durchlaß durch eine Vielzahl von Leitungen (8) gebildet wird, die sich wenigstens ungefähr parallel zueinander erstrecken und dadurch, da die Leitungen (8) winkelmäßig gleich verteilt um die Längsachse (10) der Struktur (3) sind.

2. Sonde nach Anspruch 1, dadurch gekennzeichnet, daß die Achsen (11) der Leitungen auf einer zylindrischen Oberfläche (12) liegen, deren Längsachse (10) sich mit der der Struktur (3) deckt.

3. Sonde nach einem der Ansprüche 1 oder 2, in der die Verdickung aufblasbar ist, dadurch gekennzeichnet, daß die Leitungen (8) im nicht aufgeblasenen Zustand der Verdickung (5) geschlossen und in deren aufgeblasenem Zustand geöffnet sind.

4. Sonde nach Anspruch 1, dadurch gekennzeichnet, daß die Verdickung (5) mit einer doppelten Wand (14, 15) ausgeführt ist und dadurch, daß die Leitungen (8) durch das Innere dieser doppelten Wand führen.

5. Sonde nach Anspruch 4, dadurch gekennzeichnet, daß die äußere Wand (15) der doppelten Wand (14, 15) wenigstens teilweise unter Druck porös ist, während die innere Wand (14) der Doppelwand (14, 15) einen aufblasbaren Ballon bildet.

6. Sonde nach Anspruch 5, dadurch gekennzeichnet, daß der mittlere Teil (15a) der äußeren Wand (15) unter Druck porös ist, wohingegen die Enden der äußeren Wand vorgeformt sind, um die Form von Wülsten (15b) aufzuweisen.

7. Sonde nach Anspruch 6, dadurch gekennzeichnet, daß sie eine Leitung (18) umfaßt, um die doppelte Wand (14, 15) mit einem Stoff zu versorgen, der dazu geeignet ist, den Mittelteil der äußeren Wand zu durchdringen.

*Fig: 1*

*Fig: 2*

*Fig: 3*

Fig. 4